# EUROPÄISCHE PATENTSCHRIFT

(11) **EP 1 200 049 B1**
(45) Veröffentlichungstag und Bekanntmachung des Hinweises auf die Patenterteilung: **21.02.2007**
(21) Anmeldenummer: 00956288.5
(22) Anmeldetag: 26.07.2000
(51) Int. Cl.: A61K 8/49, A61Q 5/10, D06P 3/02, D06P 1/32

(54) **MITTEL ZUM FÄRBEN VON KERATINHALTIGEN FASERN**
AGENT FOR DYING KERATIN FIBERS
AGENTS POUR COLORER DES FIBRES À BASE DE KÉRATINE

(30) Priorität: 05.08.1999 DE 19936912
(43) Veröffentlichungstag der Anmeldung: 02.05.2002
(73) Patentinhaber: Henkel Kommanditgesellschaft auf Aktien, 40589 Düsseldorf (DE)
(72) Erfinder: MÖLLER, Hinrich, D-40789 Monheim (DE); OBERKOBUSCH, Doris, D-40591 Düsseldorf (DE); HÖFFKES, Horst, D-40595 Düsseldorf (DE)
(86) Internationale Anmeldenummer: PCT/EP2000/007164
(87) Internationale Veröffentlichungsnummer: WO 2001/010398

(56) Entgegenhaltungen:
- DE-A- 19 745 356
- DE-A- 19 810 887
- DE-A- 19 859 750
- DE-A- 19 859 809

## Beschreibung

Die Erfindung betrifft ein Mittel zum Färben von keratinhaltigen Fasern, insbesondere menschlichen Haaren, das aromatische und/oder heteroaromatische Aldehyde oder Ketone enthält, die Verwendung dieser aromatischen und/oder heteroaromatischen Aldehyde oder Ketone als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamina-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die Verwendung des unten näher beschriebenen aromatischen und/oder heteroaromatischen Aldehyde oder Ketone zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel 1 dargestellten aromatischen und oder heteroaromatischen Aldehyde oder Ketone sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend mindestens einen aromatischen und/oderheteroaromatischen Aldehyd oder ein Keton mit der Formel I, 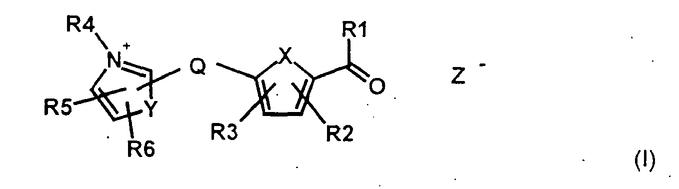 in der
R¹ ein Wasserstoffatom, eine C₁₋₄-Alkyl-, eine Aryl- oder Heteroarylgruppe,
R², R³ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R² und R³ gemeinsam einen ankondensierten aromatischen Ring bilden können, R⁴ eine C₁₋₄-Alkyl-, C₁₋₄-Alkenyl-, eine Aryl-, Aralkyl-, C₁₋₄-Carboxyalkyl- oder C₁₋₄-Sulfoalkyl-gruppe,
R⁵, R⁶ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R⁵ und R⁶ gemeinsam einen ankondensierten aromatischen Ring bilden können,
Q eine Methylen- oder Vinylengruppe bedeutet,
Y und X ein durch eine Aralkyl- oder Arylgruppe substituiertes Stickstoffatom, ein Sauerstoffatom, ein Schwefelatom oder eine gegebenenfalls substituierte Vinylengruppe bedeuten und
Z ein Anion wie Halogenid, C₁₋₄-Alkylsulfat, C₁₋₄-Alkansulfonat, Arensulfonat, C₁₋₄-Perfluoralkansulfat, Perhalogenat, Sulfat, Hydrogensulfat, Carboxylat, Tetrafluorborat, Hexafluorphosphat oder Tetrachlorzinkat bedeutet,
wobei die Bindung zum quaternierten Heterocyclus vorzugsweise in der 2-Stellung und für Y = Vinylen auch in der 4-Stellung fixiert ist.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Vorzugsweise sind die Verbindungen mit der Formel I ausgewählt aus den Vorzugsweise sind die Verbindungen mit der Formel I ausgewählt den 1-Methyl-4-[2-(4-formylphenyl)-ethenyl]-pyridinium-, 1-Methyl-2-[2-(4-formylphenyl)-ethenyl]-pyridinium-, 1-Methyl-4-[2-(4-acetylphenyl)-ethenyl]-pyridinium-, 1-Benzyl-4-[2-(4-formylphenyl)-ethenyl]-pyridinium-, 1-Methyl-4-[2-(4-formylphenyl)-ethenyl]-chinolinium-, 1-Methyl-2-[2-(4-formylphenyl)-ethenyl]-chinolinium-, 1-Methyl-2-[2-(5-formyl-2-furyl)-ethenyl]-chinolinium-, 1-Methyl-2-[2-(5-formyl-2-thienyl)-ethenyl]-chinolinium-, 1-Methyl-2-[2-(4-formylphenyl)-ethenyl]-benzothiazolinium-, 1,3-Dimethyl-2-[2-(4-formylphenyl)-ethenyl]-benzimidazolinium-, 1,3-Dimethyl-2-[2-(4-formylphenyl)-ethenyl]-imidazolinium-Salzen, insbesondere den Trifluormethansulfonaten, Benzolsulfonaten, p-Toluolsulfonaten, Methansulfonaten, Perchloraten, Sulfaten, Chloriden, Bromiden, Iodiden, Hexafluorphosphaten, Tetrachlorzinkaten, Tetrafluorboraten sowie deren beliebigen Gemische.

Diese Substanzen sind zum großen Teil literaturbekannt oder im Handel erhältlich oder nach bekannten Syntheseverfahren herstellbar.

Die voranstehend genannten aromatischen und/oder heteroaromatischen Aldehyde oder Ketone mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

Färbemittel, die als färbende Komponente die aromatischen und/oder heteroaromatischen Aldehyde oder Ketone mit der Formel I allein enthalten, werden bevorzugt für Färbungen im Grün- und Gelbbereich eingesetzt. Färbungen mit noch erhöhter Brillanz und verbesserten Echtheitseigenschaften (Lichtechtheit, Waschechtheit, Reibechtheit) über einen weiten Nuancenbereich von gelb über gelbbraun, orange, braunorange, mittelbraun, dunkelbraun, violett, dunkelviolett bis hin zu blauschwarz und schwarz werden erzielt, wenn die erfindungsgemäß eingesetzten Verbindungen mit der Formel I gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder CH-aktiven Verbindungen, verwendet werden. Dies sind einerseits Verbindungen, die für sich alleine keratinhaltige Fasern nur schwach färben und erst gemeinsam mit den Verbindungen der Formel I brillante Färbungen ergeben. Andererseits sind darunter aber auch Verbindungen, die bereits als Oxidationsfarbstoffvorprodukte eingesetzt werden.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene aromatische und/oderheteroaromatische Aldehyde oder Ketone der Formel I gemeinsam zum Einsatz kommen; ebenso können auch mehrere verschiedene Verbindungen der Komponente B gemeinsam verwendet werden.

Geeignete Verbindungen mit primärer oder sekundärer Aminogruppe als Komponente B sind z. B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, - phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 3,4-Methylendioxy-, 5-(2-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Nitrile, Aniline, insbesondere Nitrogruppen-haltige Aniline, wie 4-Nitroanitin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 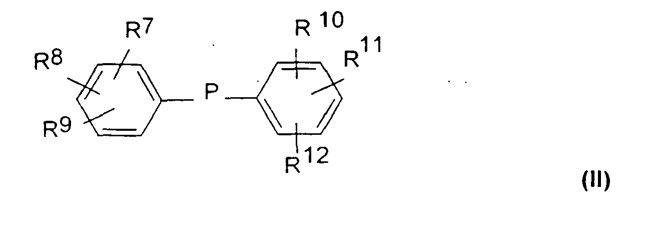 in der R⁷ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht, R⁸, R⁹, R¹⁰, R¹¹ und R¹² für Wasserstoff, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und
P für eine direkte Bindung, eine gesättigte oder ungesättigte, ggf. durchHydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

B-(CH₂-A-CH₂-B')ₒ (III)

in der
A eine direkte Bindung, eine CH₂- oder CHOH-Gruppe bedeutet,
B und B' unabhängig voneinander für ein Sauerstoffatom, eine NR¹³-Gruppe, worin R¹³ Wasserstoff, eine C₁₋₄-Alkyl- oder eine Hydroxy-C₁₋₄-alkylgruppe bedeutet, die Gruppe O-(CH₂)ₚ-NH oder NH-(CH₂)ₚ'-O, worin p und p' 2 oder 3 sind, stehen und
o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorind, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, 1,2-Dimethyl-naphtho[1,2-d]thiazofium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

Die Verbindungen der Komponente B werden besonders bevorzugt ausgewählt aus der Gruppe bestehend aus N-(2-Hydroxyethyl)-N-ethyl-, 2-Chlor-p-phenylendiamin, N,N-Bis-(2-hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie deren mit vorzugsweise anorganischen Säuren gebildeten physiologisch verträglichen Salze.

Auf die Anwesenheit von Oxidationsmitteln, z. B. H₂O₂, kann dabei verzichtet werden. Es kann jedoch u. U.. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis-(2'-hydroXyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel inwasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen: Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39.26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie .Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammonium-chlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten könditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Norrienklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinyipyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/isobornylacrylat-Copolymere. Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein weiterer Gegenstand der vorliegenden Erfindung betrifft die Verwendung von aromatischen und/oder heteroaromatischen Aldehyden oder Ketonen mit der Formel I, 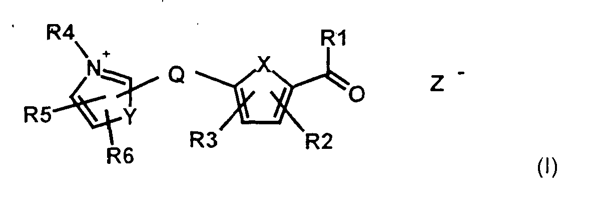 in der
R¹ ein Wasserstoffatom, eine C₁₋₄-Alkyl-, eine Aryl- oder Heteroarylgruppe,
R², R³ ein Wasserstoff-, Halogenatom, eine C₁₋₄-Alkyl-, C₁₋₄-Alkoxy- oder C₁₋₄-Hydroxyalkoxygruppe, eine Hydroxy-. Nitro, Aminogruppe, die durch C₁₋₄-Alkylgruppen substituiert sein kann, die gemeinsam mit dem Stickstoffatom einen heterocyclischen Ring bilden können, wobei auch die beiden Reste R² und R³ gemeinsam einen ankondensierten aromatischen Ring bilden können, R⁴ eine C₁₋₄-Alkyl-, C₁₋₄-Alkenyl-, eine Aryl-, Aralkyl-, C₁₋₄-Carboxyalkyl- oder C₁₋₄-Sulfoalkylgruppe,
R⁵, R⁶ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R⁵ und R⁶ gemeinsam einen ankondensierten aromatischen Ring bilden können, .
Q eine Methylen- oder Vinylengruppe bedeutet,
Y und X ein durch eine Aralkyl- oder Arylgruppe substituiertes Stickstoffatom, ein Sauerstoffatom, ein Schwefelatom oder eine gegebenenfalls substituierte Vinylengruppe bedeuten und
Z⁻ ein Anion wie Halogenid, C₁₋₄-Alkylsulfat, C₁₋₄-Alkansulfonat, Arensulfonat, C₁₋₄-Perfluoralkansulfat, Perhalogenat, Sulfat, Hydrogensulfat, Carboxylat, Tetrafluorborat, Hexafluorphosphat oder Tetrachlorzinkat bedeutet,
wobei die Bindung zum quaternierten Heterocyclus vorzugsweise in der 2-Stellung und für Y = Vinylen auch in der 4-Stellung fixiert ist,
als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend,
A) mindestens einen aromatischen und/oder heteroaromatischen Aldehyd oder ein Keton mit der Formel I, 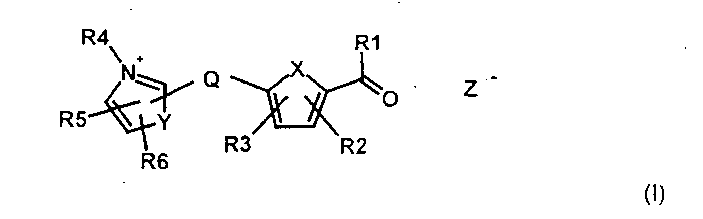 in der
   R¹ ein Wasserstoffatom, eine C₁₋₄-Alkyl-, eine Aryl- oder Heteroarylgruppe,
   R², R³ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R² und R³ gemeinsam einen ankondensierten aromatischen Ring bilden können, R4 eine C₁₋₄-Alkyl-, C₁₋₄-Alkenyl-, eine Aryl-, Aralkyl-, C₁₋₄-Carbocyalkyl- oder C₁₋₄-Sulfoalkylgruppe,
   Q eine Methylen- oder Vinylengruppe bedeutet,
   R⁵, R⁶ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R⁵ und R⁶ gemeinsam einen ankondensierten aromatischen Ring bilden können,
   Y und X ein durch eine Aralkyl- oder Arylgruppe substituiertes Stickstoffatom, ein Sauerstoffatom, ein Schwefelatom oder eine gegebenenfalls substituierte Vinylengruppe bedeuten und.
   Z ein Anion wie Halogenid, C₁₋₄-Alkylsulfat, C₁₋₄-Alkansulfonat, Arensulfonat, C₁₋₄-Perfluoralkansulfat, Perhalogenat, Sulfat, Hydrogensulfat, Carboxylat, Tetrafluorborat, Hexafluorphosphat oder Tetrachlorzinkat bedeutet,
   wobei die Bindung zum quaternierten Heterocyclus vorzugsweise in der 2-Stellung und für Y = Vinylen auch in der 4-Stellung fixiert ist, und
B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, aromatischen Hydroxyverbindungen, und/oder mindestens eine CH-aktive Verbindung,
sowie übliche kosmetische Inhaltsstoffe, auf die keratinhaltigen Fasern aufgebracht, einige Zeit, üblicherweise ca 30 Minuten, auf der Faser belassen und anschließend wieder ausgespült oder mit einem Shampoo ausgewaschen wird.

Die aromatischen und/oder heteroaromatischen Aldehyde oder Ketonen der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die aromatischen und/oder heteroaromatischen Aldehyde oder Ketone der Formel I und die Verbindungen der Komponente B können entweder getrennt oder zusammen gelagert werden, entweder in einer flüssigen bis pastösen Zubereitung (wäßrig oderwasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellungsbeispiele

### 1-Methyl-4-[2-(4-formylphenyl)-ethenyl]-pyridinium-methylsulfat

Zu der gekühlten Lösung von 18,8 g (200 mmol) γ-Picolin in 50 ml Methanol wurden bei 0°C langsam 25,2 g (200mmol) Dimethylsulfat getropft und nach einer Std. Rühren bei dieser Temperatur und Erwärmen auf Raumtemperatur wurden 80,4 g (600 mmol) Terephthalaldehyd und 2,8 ml Piperidin zugegeben. Anschließend wurde 5 Std. zum Sieden erhitzt, die Mischung heiß filtriert und die Mutterlauge in eine Mischung aus 300 ml Ethanol und 100 ml Aceton eingerührt und über Nacht stehen gelassen. Der ausgefallene Niederschlag wurde abgesaugt, zuerst mit Ethanol, dann mit Aceton gewaschen und getrocknet. Es wurden 44,5 g (66,3% d. Th.) eines gelblichen kristallinen Produkts vom Schmp. 206-214°C erhalten.

### 1-Methyl-4-[2-(4-formylphenyl)-ethenyl]-chinolinium-methylsulfat

wurde analog erhalten: Schmp. 179-180°C

### Herstellung einer Färbelösung

Es wurde je eine Aufschlämmung von 5 mmol eines aromatischen und/oder heteroaromatischen Aldehyds öder Ketons der Formel I, ggf. 5 mmol eines Oxidationsfarbstoffvorproduktes der Komponente B und 5 mmol Natriumacetat und einen Tropfen einer 20-%igen Fettalkylethersulfat-Lösung in 50 ml Wasser bei 60°C hergestellt. Die Aufschlämmungen wurden nach Abkühlen auf 30°C miteinander vermischt und mit verdünnter NaOH oder Salzsäure auf einen pH-Wert von 9 eingestellt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:

| | |
|---|---|
| - : | keine oder eine sehr blasse Ausfärbung |
| (+) : | schwache Intensität |
| + : | mittlere Intensität |
| +(+) : | mittlere bis starke Intensität |
| ++ : | starke Intensität |
| ++(+) : | starke bis sehr starke Intensität |
| +++ : | sehr starke Intensität |

**Tabelle 1**

| **Ausfärbungen mit 1-Methyl-4-[2-(4-formylphenyl)-ethenyl]-pyridinium-methylsulfat** | | |
|---|---|---|
| **Komponente B** | **Farbe** | **Intensität** |
| -- | hellgelb | + |
| 2,5-Diaminotoluol x H₂SO₄ | braunorange | ++ |
| 2,4,5,6-Tetraaminopyrimidin x H₂SO₄ | leuchtend orange | ++(+) |
| 2-Methylamino-3-amino-6-methoxypyridin x 2 HCl | rostrot | ++(+) |
| 2-(2,5-Diaminophenyl)-ethanol x H₂SO₄ | braunorange | ++ |
| 2-Aminomethyl-4-aminophenol x 2 HCl | goldgelb | ++(+) |
| N,N-Bis-(2-hydroxyethyl)-p-phenylendiamin x | dunkel | ++ |
| H₂SO₄ | orangebraun | |
| 4,4'-Diaminodiphenylamin x H₂SO₄ | rotviolett | ++ |

**Tabelle 2**

| **Ausfärbungen mit 1-Methyl-4-[2-(4-formylphenyl)-ethenyl]-chinolinium-methylsulfat** | | |
|---|---|---|
| **Komponente B** | **Farbe** | **Intensität** |
| -- | gelbgrün | ++ |
| 2,5-Diaminotoluol x H₂SO₄ | orangebraun | ++ |
| 2,4,5,6-Tetraaminopyrimidin x H₂SO₄ | braunrot | ++(+) |
| 1,8-Bis-(2,5-diaminophenoxy)-3,6-dioxaoctan x 4 HCl | mittelbraun | ++ |
| 2-Methylamino-3-amino-6-methoxypyridin x 2 HCl | rotviolett | +++ |
| 2-(2,5-Diaminophenyl)-ethanol x H₂SO₄ | oranagebraun | ++ |
| 2-Aminomethyl-4-aminophenol x 2 HCl | oranage | ++ |
| N,N-Bis-(2-hydroxyethyl)-p-phenylendiamin x H₂SO₄ | rotviolett | +++ |
| 4,4'-Diaminodiphenylamin x H₂SO₄ | braunorange | +(+) |
| 3,5-Diamino-2,6-dimethoxy-pyridin x 2 HCl | schwarz | +++ |

## Patentansprüche

1. Mittel zum Färben von keratinhaltigen Fasern, insbesondere menschlichen Haaren, enthaltend als färbende Komponente mindestens einen aromatischen und/oder heteroaromatischen Aldehyd oder ein Keton mit der Formel I, 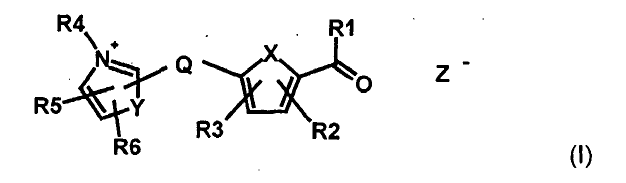 in der
R¹ ein Wasserstoffatom, eine C₁₋₄-Alkyl-, eine Aryl- oder Heteroarylgruppe,
R², R³ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R² und R³ gemeinsam einen ankondensierten aromatischen Ring bilden können, R⁴ eine C₁₋₄-Alkyl-, C₁₋₄-Alkenyl-, eine Aryl-, Aralkyl-, C₁₋₄-Carboxyalkyl- oder C₁₋₄-Sulfoalkylgruppe,
R⁵, R⁶ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R⁵ und R⁶ gemeinsam einen ankondensierten aromatischen Ring bilden können,
Q eine Methylen- oder Vinylengruppe bedeutet,
Y und X ein durch eine Aralkyl- oder Arylgruppe substituiertes Stickstoffatom, ein Sauerstoffatom, ein Schwefelatom oder eine gegebenenfalls substituierte Vinylengruppe bedeuten und
Z⁻ ein Anion wie Halogenid, C₁₋₄-Alkylsulfat, C₁₋₄-Alkansulfonat, Arensulfonat, C₁₋₄-Perfluoralkansulfat, Perhalogenat, Sulfat, Hydrogensulfat, Carboxylat, Tetrafluorborat, Hexafluorphosphat oder Tetrachlorzinkat bedeutet,
wobei die Bindung zum quaternierten Heterocyclus vorzugsweise in der 2-Stellung und für Y = Vinylen auch in der 4-Stellung fixiert ist.

2. Mittel nach Anspruch 1, **dadurch gekennzeichnet, daß als** Verbindungen mit der Formel I 1-Methyl-4-[2-(4-formylphenyl)-ethenyl]-pyridinium-, 1-Methyl-2-[2-(4-formylphenyl)-ethenyl]-pyridinium-, 1-Methyl-4-[2-(4-acetylphenyl)-ethenyl]-pyridinium-, 1-Benzyl-4-[2-(4-formylphenyl)-ethenyl]-pyridinium-, 1-Methyl-4-[2-(4-formylphenyl)-ethenyl]-chinolinium-, 1-Methyl-2-[2-(4-formylphenyl)-ethenyl]-chinolinium-, 1-Methyl-2-[2-(5-formyl-2-furyl)-ethenyl]-chinolinium-, 1-Methyl-2-[2-(5-formyl-2-thienyl)-ethenyl]-chinolinium-, 1-Methyl-2-[2-(4-formylphenyl)-ethenyl]-benzothiazolinium-, 1,3-Dimethyl-2-[2-(4-formylphenyl)-ethenyl]-benzimidazolinium-, 1,3-Dimethyl-2-[2-(4-formylphenyl)-ethenyl]-imidazolinium-Salze, insbesondere die Trifluormethansulfonate, Benzolsulfonate, p-Toluolsulfonate, Methansulfonate, Perchlorate, Sulfate, Chloride, Bromide, Iodide, Tetrachlorzinkate, Tetrafluorboraten sowie deren beliebigen Gemische eingesetzt werden.

3. Mittel nach einem der Ansprüche 1 bis 2, **dadurch gekennzeichnet, daß** die aromatischen und/oder heteroaromatischen Aldehyde oder Ketone der Formel I in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, daß** es zusätzlich mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder mindestens eine CH-aktive Verbindung, enthält.

5. Mittel nach Anspruch 4, **dadurch gekennzeichnet, daß** die weitere Verbindung ausgewählt ist aus
primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, -phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 2-Hydroxymethyl-4-aminophenol, 2-Diethylaminomethyl-4-aminophenol, 2-Dimethylaminomethyl-4-aminophenol, 2,6-Dichlor-4-aminophenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxybenzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulforisäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Nitrile, Aniline, insbesondere Nitrogruppen-haltige Aniline, wie 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2,5-diarninophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamin-trihydrochlorid,
stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure, und
CH-aktiven Verbindungen ausgewählt aus der Gruppe, bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin); 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Ethyl-2-chinaldinium-iodid, 1,4-Dimethylchinolinium-iodid, 1-Methyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

6. Mittel nach Anspruch 5, **dadurch gekennzeichnet, daß** die weitere Verbindung ausgewählt ist aus der Gruppe bestehend aus N-(2-Hydroxyethyl)-N-ethyl-, 2-Chlor-p-phenylendiamin, N, N-Bis-(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie jeweils aus den vorzugsweise mit anorganischen Säuren gebildeten physiologisch verträglichen Salzen dieser Verbindungen.

7. Mittel nach einem der Ansprüche 1 bis 6, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

8. Mittel nach einem der Ansprüche 1 bis 7, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

9. Mittel nach einem der Ansprüche 1 bis 8, **dadurch gekennzeichnet, daß** es Oxidationsmittel, insbesondere H₂O₂, in einer Menge von 0,01 bis 6 Gew.-%, bezogen auf die Anwendungslösung, enthält.

10. Mittel nach einem der Ansprüche 1 bis 9, **dadurch gekennzeichnet, daß** es anionische, zwitterionische oder nichtionische Tenside enthält.

11. Verwendung von aromatischen und/oder heteroaromatischen Aldehyden oder Ketonen mit der Formel I, 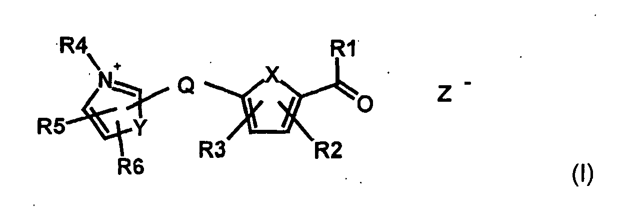 in der
R¹ ein Wasserstoffatom, eine C₁₋₄-Alkyl-, eine Aryl- oder Heteroarylgruppe,
R², R³ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R² und R³ gemeinsam einen ankondensierten aromatischen Ring bilden können, R4 eine C₁₋₄-Alkyl-, C₁₋₄-Alkenyl-, eine Aryl-, Aralkyl-, C₁₋₄-Carboxyalkyl- oder C₁₋₄-Sulfoalkylgruppe,
R⁵, R⁶ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R⁵ und R⁶ gemeinsam einen ankondensierten aromatischen Ring bilden können,
Q eine Methylen- oder Vinylengruppe bedeutet,
Y und X ein durch eine Aralkyl- oder Arylgruppe substituiertes Stickstoffatom, ein Sauerstoffatom, ein Schwefelatom oder eine gegebenenfalls substituierte Vinylengruppe bedeuten und
Z⁻ ein Anion wie Halogenid, C₁₋₄-Alkylsulfat, C₁₋₄-Alkansulfonat, Arensulfonat, C₁₋₄-Perfluoralkansulfat, Perhalogenat, Sulfat, Hydrogensulfat, Carboxylat, Tetrafluorborat, Hexafluorphosphat oder Tetrachforzinkat bedeutet,
wobei die Bindung zum quaternierten Heterocyclus vorzugsweise in der 2-Stellung und für Y = Vinylen auch in der 4-Stellung fixiert ist,
als eine färbende Komponente in Oxidationshaarfärbemitteln.

12. Verfahren zum Färben von keratinhaltigen Fasern, insbesondere menschlichen Haaren, worin ein Färbemittel, enthaltend
A) mindestens einen aromatischen und/oder heteroaromatischen Aldehyd oder ein Keton mit der Formel I, 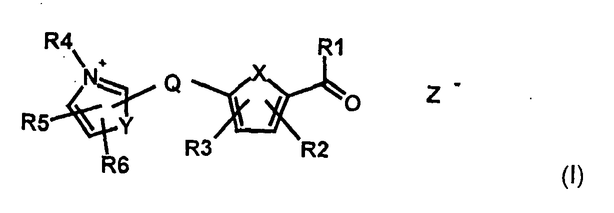 in der
R¹ ein Wasserstoffatom, eine C₁₋₄-Alkyl-, eine Aryl- oder Heteroarylgruppe,
R², R³ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R² und R³ gemeinsam einen ankondensierten aromatischen Ring bilden können, R⁴ eine C₁₋₄-Alkyl-, C₁₋₄-Alkenyl-, eine Aryl-, Aralkyl-, C₁₋₄-Carboxyalkyl- oder C₁₋₄-Sulfoalkylgruppe,
R⁵, R⁶ ein Wasserstoff-, Halogenatom, eine C₁₋₄-Alkyl-, C₁₋₄-Alkoxy- oder C₁₋₄-Hydroxyalkoxygruppe, eine Hydroxy-, Nitro-, Aminogruppe, die durch C₁₋₄-Alkylgruppen substituiert sein kann, die gemeinsam mit dem Stickstoffatom einen heterocyclischen Ring bilden können, wobei auch die beiden Reste R⁵ und R⁶ gemeinsam einen ankondensierten aromatischen Ring bilden können,
Q eine Methylen- oder Vinylengruppe bedeutet,
Y und X ein durch eine Aralkyl- oder Arylgruppe substituiertes Stickstoffatom, ein Sauerstoffatom, ein Schwefelatom oder eine gegebenenfalls substituierte Vinylengruppe bedeuten und
Z' ein Anion wie Halogenid, C₁₋₄-Alkylsulfat, C₁₋₄-Alkansulfonat, Arensulfonat, C₁₋₄-Perfluoralkansulfat, Perhalogenat, Sulfat, Hydrogensulfat, Carboxylat, Tetrafluorborat, Hexafluorphosphat oder Tetrachlorzinkat bedeutet,
wobei die Bindung zum quaternierten Heterocyclus vorzugsweise in der 2-Stellung und für Y = Vinylen auch in der 4-Stellung fixiert ist,
B) mindestens eine Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, und/oder mindestens eine CH-aktive Verbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Agent for dyeing keratin fibres, in particular human hair, comprising, as colouring component, at least one aromatic and/or heteroaromatic aldehyde or a ketone with the formula I, 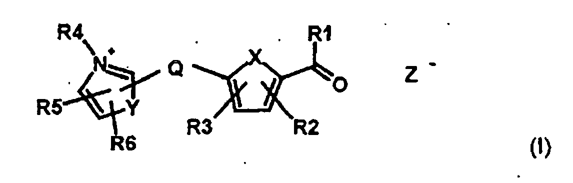 in which R¹ is a hydrogen atom, a C₁₋₄-alkyl group, an aryl group or heteroaryl group,
R², R³ are a hydrogen atom, halogen atom, a C₁₋₄-alkyl group, C₁₋₄-alkoxy group or C₁₋₄-hydroxy-alkoxy group, a hydroxy group, nitro group, amino group, which may be substituted by C₁₋₄-alkyl groups, which, together with the nitrogen atom, can form a heterocyclic ring, where also.the two radicals R² and R³ can together form a fused-on aromatic ring, R⁴ is a C₁₋₄-alkyl group, C₁₋₄-alkenyl group, an aryl group, aralkyl group, C₁₋₄-carboxyalkyl group or C₁₋₄-sulphoalkyl group,
R⁵, R⁶ are a hydrogen atom, halogen atom, a C₁₋₄-alkyl group, C₁₋₄-alkoxy group or C₁₋₄-hydroxy-alkoxy group, a hydroxy group, nitro group, amino group, which may be substituted by C₁₋₄-alkyl groups, which, together with the nitrogen atom, can form a heterocyclic ring, where also the two radicals R⁵ and R⁶ can together form a fused-on aromatic ring,
Q is a methylene or vinylene group,
Y and X are a nitrogen atom substituted by an aralkyl or aryl group, an oxygen atom, a sulphur atom or an optionally substituted vinylene group and
Z⁻ is an anion such as halide, C₁₋₄-alkyl sulphate, C₁₋₄-alkanesulphonate, arenesulphonate, C₁₋₄-perfluoroalkane sulphate, perhalogenate, sulphate, hydrogen sulphate, carboxylate, tetrafluoroborate, hexafluorophosphate or tetrachlorozincate,
where the bond to the quaternized heterocycle is preferably fixed in the 2-position and for Y = vinylene also in the 4-position.

2. Agent according to Claim 1, **characterized in that** the compounds with the formula I used are 1-methyl-4-[2-(4-formylphenyl)ethenyl]pyridinium, 1-methyl-2-[2-(4-formylphenyl)ethenyl]pyridinium, 1-methyl-4-[2-(4-acetylphenyl)ethenyl]pyridinium, 1-benzyl-4-[2-(4-formylphenyl)ethenyl]pyridinium, 1-methyl-4-[2-(4-formylphenyl)ethenyl]quinolinium, 1-methyl-2-[2-(4-formylphenyl)ethenyl]quinolinium, 1-methyl-2-[2-(5-formyl-2-furyl)ethenyl]-quinolinium, 1-methyl-2-[2-(5-formyl-2-thienyl)-ethenyl]quinolinium, 1-methyl-2-[2-(4-formylphenyl)ethenyl]benzothiazolinium, 1,3-dimethyl-2-[2-(4-formylphenyl)ethenyl]benzimidazolinium, 1,3-dimethyl-2-[2-(4-formylphenyl)ethenyl]-imidazolinium salts, in particular the trifluoromethanesulphonates, benzenesulphonates, p-toluene-sulphonates, methanesulphonates, perchlorates, sulphates, chlorides, bromides, iodides, tetrachlorozincates, tetrafluoroborates, and any mixtures thereof.

3. Agent according to one of Claims 1 to 2, **characterized in that** the aromatic and/or heteroaromatic aldehydes or ketones of the formula I are present in an amount of from 0.03 to 65 mmol, in particular from 1 to 40 mmol, based on 100 g of the total colorant.

4. Agent according to one of Claims 1 to 3, **characterized in that** it additionally comprises at least one compound with primary or secondary amino group or hydroxy group chosen from primary or secondary aromatic amines, nitrogen-containing heterocyclic compounds and aromatic hydroxy compounds, and/or at least one CH-active compound.

5. Agent according to Claim 4, **characterized in that** the further compound is chosen from
primary or secondary amines from the group consisting of N-(2-hydroxyethyl)-N-ethyl, N-(2-methoxyethyl)-, 2,3-, 2,4-, 2-5-dichloro-p-phenylenediamine, 2-chloro-p-phenylenediamine, N,N-bis(2-hydroxyethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)ethanol, 2,5-diaminotoluene, -phenol, -phenetol, 4-methylamino-, 3-amino-4-(2'-hydroxy-ethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 5-(2-hydroyethylamino)-4-methoxy-2-methyl-, 4-amino-2-aminomethylphenol, 2-hydroxymethyl-4-aminophenol, 2-diethylaminomethyl-4-aminophenol, 2-dimethylaminomethyl-4-aminophenol, 2,6-dichloro-4-aminophenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzene-sulphonic acid, 3-amino-4-hydroxybenzenesulphonic acid, 4-amino-3-hydroxynaphthalenesulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechin sulphate, 4,6-diaminopyrogallol dihydrochloride, 3,5-diamino-4-hydroxypyrocatechin sulphate, aromatic nitriles, anilines, in particular nitro-group-containing anilines, such as 4-nitroaniline, 4-nitro-1,3-phenylenediamine, 2-nitro-4-amino-1-(2-hydroxyethylamino)benzene, 2-nitro-1-amino-4-[bis(2-hydroxyethyl)amino]benzene, 4-amino-2-nitrodiphenylamine-2'-carboxylic acid, 2-amino-6-chloro-4-nitrophenol, 1-amino-5-chloro-4-(2-hydroxyethylamino)-2-nitrobenzene, aromatic anilines and phenols with a further aromatic radical, such as 4,4'-diaminostilbene dihydrochloride, 4,4'-diaminostilbene-2,2'-disulphonic acid, Na salt, 4,4'-diaminodiphenylmethane, sulphide, sulphoxide, -amine, 4,4'-diaminodiphenylamine-2-sulphonic acid, 4,4'-diaminobenzophenone, diphenyl ether, 3,3', 4,4'-tetraaminodiphenyl tetrahydrochloride, 3,3',4,4'-tetraaminobenzophenone, 1,3-bis(2,4-diaminophenoxy)propane tetrahydrochloride, 1,8-bis(2,5-diaminophenoxy)-3,6-dioxaoctane tetrahydrochloride, 1,3-bis(4-aminophenylamino)propane, -2-propanol, 1,3-bis[N-(4-aminophenyl)-2-hydroxyethylamino]-2-propanol, N,N-bis[2-(4-aminophenoxy)-ethyl]methylamine trihydrochloride,
nitrogen-containing heterocyclic compounds chosen from the group consisting of 2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethylpyridine, 4,5,6-triamino-, 2-hydroxy-4,5,6-triamino-, 4-hydroxy-2,5,6-triamino-, 2,4,5,6-tetraamino-, 2-methylamino-4,5,6-triamino-, 2,4-, 4,5-diamino-, 2-amino-4-methoxy-6-methylpyrimidine, 2,3,4-trimethylpyrrole, 2,4-dimethyl-3-ethylpyrrole, 3,5-diaminopyrazole, -1,2,4-triazole, 3-amino-, 3-amino-5-hydroxypyrazole, 2-, 3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2,5-dihydroxy-4-morpholinoaniline, and indole and indoline derivatives, such as 4-, 5-, 6-, 7-aminoindole, 4-, 5-, 6-, 7-hydroxyindole, 5,6-dihydroxyindole, 5,6-dihydroxyindoline and 4-hydroyxindoline, and in each case from the physiologically compatible salts of these compounds formed with preferably inorganic acids,
aromatic hydroxy compounds chosen from the group consisting of 2-, 4-, 5-methylresorcinol, 2,5-dimethylresorcinol, resorcinol, 3-methoxyphenol, pyrocatechin, hydroquinone, pyrogallol, phloroglucine, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulphonic acid, 3-6-dihydroxy-2,7-naphthalenesulphonic acid, and CH-active compounds chosen from the group consisting of 1,2,3,3-tetramethyl-3H-indolium iodide, 1,2,3,3-tetramethyl-3H-indolium p-toluenesulphonate, 1,2,3,3-tetramethyl-3H-indolium methanesulphonate, Fischer's base (1,3,3-trimethyl-2-methyleneindoline); 2,3-dimethylbenzothiazolium iodide, 2,3-dimethyl-benzothiazolium p-toluenesulphonate, 1,2-dimethylnaphtho[1,2-d]thiazolium p-toluenesulphonate, 1-ethyl-2-methylnaphtho[1,2-d]thiazolium p-toluenesulphonate, rhodanine, rhodanine-3-acetic acid, 1-ethyl-2-quinaldinium iodide, 1,4-dimethylquinolinium iodide, 1-methyl-2-quinaldinium iodide, 1,4-dimethylquinolinium iodide, barbituric acid, thiobarbituric acid, 1,3-dimethylthiobarbituric acid, 1,3-diethylthiobarbituric acid, diethylthiobarbituric acid, oxindole, 3-indoxyl acetate, coumaranone and 1-methyl-3-phenyl-2-pyrazolinone.

6. Agent according to Claim 5, **characterized in that** the further compound is chosen from the group consisting of N-(2-hydroxethyl)-N-ethyl-, 2-chloro-p-phenylenediamine, N,N-bis(2-hydroxyethyl)-p-phenylendiamine, 4-aminophenol, p-phenylenediamine, 2-(2,5-diaminophenyl)ethanol, 2,5-diaminotoluene, 3,4-methylenedioxyaniline, 2-amino-4-(2-hydroxyethylamino)anisole, 2-(2,4-diaminophenoxy)ethanol, 3-amino-2,4-dichloro-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 2-aminomethyl-4-aminophenol, 2-diethylaminomethyl-4-aminophenol, 2-dimethylaminomethyl-4-aminophenol, 2,6-dichloro-4-aminophenol, 2-hydroxymethyl-4-aminophenol, 3,4-methylenedioxyphenol, 3,4-diaminobenzoic acid, 2,5-diamino-, 2-dimethylamino-5-amino-, 3-amino-2-methylamino-6-methoxy-, 2,3-diamino-6-methoxy-, 3,5-diamino-2,6-dimethoxy-, 2,6-dihydroxy-3,4-dimethylpyridine, 2-hydroxy-4,5,6-triamino-, 4-hydroxy-2,5,6-triamino-, 2,4,5,6-tetraamino-, 2-methylamino-4,5,6-triaminopyrimidine, 3,5-diaminopyrazole, 3-amino-5-hydroxypyrazole, 5, 6-dihydroxyindole and 5,6-dihydroxyindoline, and in each case of the physiologically compatible salts of these compounds formed preferably with inorganic acids.

7. Agent according to one of Claims 1 to 6, **characterized in that** it comprises direct dyes from the group of nitrophenylenediamines, nitroaminophenols, anthraquinones or indophenols preferably in an amount of from 0.01 to 20% by weight, based on the total colorant.

8. Agent according to one of Claims 1 to 7, **characterized in that** ammonium salts or metal salts chosen from the group of formates, carbonates, halides, sulphates, butyrates, valeriates, capronates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

9. Agent according to one of Claims 1 to 8, **characterized in that** it comprises oxidizing agents, in particular H₂O₂, in an amount of from 0.01 to 6% by weight, based on the application solution.

10. Agent according to one of Claims 1 to 9, **characterized in that** it comprises anionic, zwitterionic or nonionic surfactants.

11. Use of aromatic and/or heteroaromatic aldehydes or ketones with the formula I 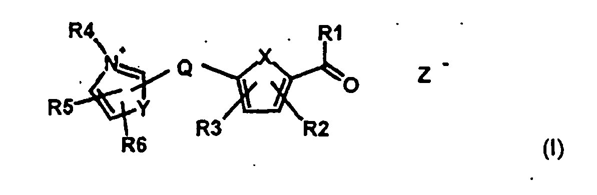 in which R¹ is a hydrogen atom, a C₁₋₄-alkyl group, an aryl group or heteroaryl group,
R ² R³ are a hydrogen atom, halogen atom, a C₁₋₄-alkyl group, C₁₋₄-alkoxy group or C₁₋₄-hydroxyalkoxy group, a hydroxy group, nitro group, amino group, which may be substituted by C₁₋₄-alkyl groups, which, together with the nitrogen atom, can form a heterocyclic ring, where also the two radicals R² and R³ can together form a fused-on aromatic ring, R⁴ is a C₁₋₄-alkyl group, C₁₋₄-alkenyl group, an aryl group, aralkyl group, C₁₋₄-carboxyalkyl group or C₁₋₄-sulphoalkyl group,
R ⁵ R⁶ are a hydrogen atom, halogen atom, a C₁₋₄-alkyl group, C₁₋₄-alkoxy group or C₁₋₄-hydroxyalkoxy group, a hydroxy group, nitro group, amino group, which may be substituted by C₁₋₄-alkyl groups, which, together with the nitrogen atom, can form a heterocyclic ring, where also the two radicals R⁵ and R⁶ can together form a fused-on aromatic ring,
Q is a methylene or vinylene group,
Y and X are a nitrogen atom substituted by an aralkyl or aryl group, an oxygen atom, a sulphur atom or an optionally substituted vinylene group and
Z⁻ is an anion such as halide, C₁₋₄-alkyl sulphate, C₁₋₄-alkanesulphonate, arenesulphonate, C₁₋₄-perfluoroalkane sulphate, perhalogenate, sulphate, hydrogen sulphate, carboxylate, tetrafluoroborate, hexafluorophosphate or tetrachlorozincate,
where the bond to the quaternized heterocycle is preferably fixed in the 2-position and for Y = vinylene also in the 4-position, as a colouring component in oxidation hair colorants.

12. Method of colouring keratin fibres, in particular human hair, in which a colorant comprising
A) at least one aromatic and/or heteroaromatic aldehyde or a ketone with the formula I, 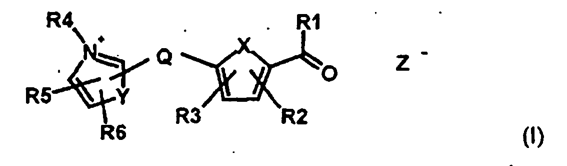
in which R¹ is a hydrogen atom, a C₁₋₄-alkyl group, an aryl group or heteroaryl group,
R², R³ are a hydrogen atom, halogen atom, a C₁₋₄-alkyl group, C₁₋₄-alkoxy group or C₁₋₄-hydroxyalkoxy group, a hydroxy group, nitro group, amino group, which may be substituted by C₁₋₄-alkyl groups, which, together with the nitrogen atom, can form a heterocyclic ring, where also the two radicals R² and R³ can together form a fused-on aromatic ring, R⁴ is a C₁₋₄-alkyl group, C₁₋₄-alkenyl group, an aryl group, aralkyl group, C₁₋₄-carboxyalkyl group or C₁₋₄-sulphoalkyl group,
R⁵, R⁶ are a hydrogen atom, halogen atom, a C₁₋₄-alkyl group, C₁₋₄-alkoxy group or C₁₋₄-hydroxyalkoxy group, a hydroxy group, nitro group, amino group, which may be substituted by C₁₋₄-alkyl groups, which, together with the nitrogen atom, can form a heterocyclic ring, where also the two radicals R⁵ and R⁶ can together form a fused-on aromatic ring,
Q is a methylene or vinylene group,
Y and X are a nitrogen atom substituted by an aralkyl or aryl group, an oxygen atom, a sulphur atom or an optionally substituted vinylene group and
Z⁻ is an anion such as halide, C₁₋₄-alkyl sulphate, C₁₋₄-alkanesulphonate, arenesulphonate, C₁₋₄-perfluoroalkane sulphate, perhalogenate, sulphate, hydrogen sulphate, carboxylate, tetrafluoroborate, hexafluorophosphate or tetrachlorozincate,
where the bond to the quaternized heterocycle is preferably fixed in the 2-position and for Y = vinylene also in the 4-position,
B) at least one compound with primary or secondary amino group or hydroxy group chosen from primary or secondary aromatic amines, nitrogen-containing heterocyclic compounds and aromatic hydroxy compounds, and/or at least one CH-active compound,
and customary cosmetic ingredients, is applied to the keratin fibres, left for some time, usually about 30 minutes, on the fibres and then rinsed out again or washed out with a shampoo.

## Revendications

1. Agent pour colorer les fibres kératiniques, en particulier les cheveux humains, contenant en tant que composant colorant au moins un aldéhyde ou une cétone aromatique et /ou hétéro aromatique de formule I 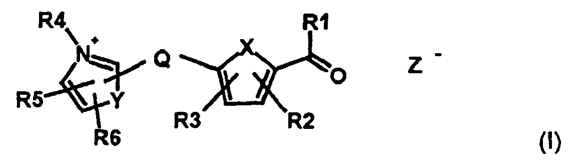 dans laquelle
R¹ représente un atome d'hydrogène, un groupe alkyle en C₁ à C₄, aryle ou hétéroaryle,
R², R³ représentent un atome d'hydrogène, un atome d'halogène, un groupe alkyle en C₁ à C₄, alcoxy en C₁ à C₄, ou hydroxyalcoxy en C₁ à C₄, un groupe hydroxy, nitro, amino, qui peut être substitué par des groupes alkyle en C₁ à C₄, qui peuvent former conjointement avec l'atome d'azote, un cycle hétérocyclique, les deux radicaux R² et R³ pouvant former également conjointement un cycle aromatique condensé, R⁴ représente un groupe alkyle en C₁ à C₄, alcényle en C₁ à C₄, aryle, aralkyle, carboxyalkyle en C₁ à C₄, ou sulfoalkyle en C₁ à C₄,
R⁵, R⁶ représentent un atome d'hydrogène, un atome d'halogène, un groupe alkyle en C₁ à C₄, alcoxy en C₁ à C₄, ou hydroxyalcoxy en C₁ à C₄, un groupe hydroxy, nitro, amino, qui peut être substitué par des groupes alkyle en C₁ à C₄, qui peuvent former conjointement avec l'atome d'azote, un cycle hétéro cyclique, les deux radicaux R⁵ et R⁶ pouvant former également conjointement un cycle aromatique condensé,
Q désigne un groupe méthylène ou vinylène,
Y et X désignent un atome d'azote substitué par un groupe aralkyle ou aryle, un atome d'oxygène, un atome de soufre, ou un groupe vinylène éventuellement substitué, et
Z- désigne un anion tel qu'un ion halogènure, sulfate d'alkyle en C₁ à C₄, alcane-sulfonate en C₁ à C₄, arènesulfonate, perfluoroalcane-sulfate en C₁ à C₄, perhalogénate, sulfate, hydrogénosulfate, carboxylate, tétrafluoroborate, hexafluorophosphate ou tétrachlorozincate,
la liaison à l'hétérocycle quaternisé étant fixée de préférence dans la position 2 et pour Y = vinylène, également dans la position 4.

2. Agent selon la revendication 1, **caractérisé en ce que** des sels de 1-méthyl-4-[2-(4-formylphényl)-éthényl]-pyridinium, de 1-méthyl-2-[2-(4-formylphényl)-éthényl]-pyridinium, de 1-méthyl-4-[2-(4-acétylphényl)-éthényl]-pyridinium, de 1-benzyl-4-[2-(4-formylphényl)-éthényl]-pyridinium, de 1-méthyl-4-[2-(4-formylphényl)-éthényl]-quinoléinium, de 1-méthyl-2-[2-(4-formylphényl)-éthényl]-quinoléinium, de 1-méthyl-2-[2-(5-formyl-2-furyl)-éthényl]-quinoléinium, de 1-méthyl-2-[2-(5-formyl-2-thiényl)-éthényl]-quinoléinium, de 1-méthyl-2-[2-(4-formylphényl)-éthényl]-benzothiazolinium, de 1,3-diméthyl-2-[2-(4-formyl-phényl)-éthényl]-benzimidazolinium, de 1,3-diméthyl-2-[2-(4-formylphényl)-éthényl]-imidazolinium, en particulier les trifluorométhanesulfonates, les benzènesulfonates, les p-toluènesulfonates, les méthanesulfonates, les perchlorates, les sulfates, les chlorures, les bromures, les iodures, les tétrachlorozincates, les tétrafluoroborates ainsi que leurs mélanges quelconques, sont mis en oeuvre en tant que composé de formule I.

3. Agent selon l'une quelconque des revendications 1 à 2, **caractérisé en ce que** les aldéhydes ou cétones aromatiques et/ou hétéroaromatiques de formule I sont contenus en une quantité de 0,03 à 65 mmoles, en particulier de 1 à 40 mmoles, par rapport à 100 g de la teinture totale.

4. Agent selon l'une quelconque des revendications 1 à 3, **caractérisé en ce qu'**il contient en outre au moins un composé avec un groupe amino primaire ou secondaire ou un groupe hydroxy, choisi parmi les amines aromatiques primaires ou secondaires, les composés hétérocycliques azotés, et les composés hydroxyaromatiques, et/ou au moins un composé à groupe CH actif.

5. Agent selon la revendication 4, **caractérisé en ce que** le composé supplémentaire est choisi parmi
les amines primaires ou secondaires issues du groupe formé par la N-(2-hydroxyéthyl)-N-éthyl-, N-(2-méthoxyéthyl)-, 2,3-, 2,4-, 2,5-dichloro-p-phénylènediamine, la 2-chloro-p-phénylènediamine, la N,N-bis-(2-hydroxyéthyl)-p-phénylènediamine, le dibromhydrate de 2,5-dihydroxy-4-morpholinoaniline, le 2-, 3-, 4-aminophénol, la o-, m-, p-phénylènediamine, le 2,4-diaminophénoxyéthanol, le 2-(2,5-diaminophényl)-éthanol, le 2,5-diaminotoluène, le 2,5-diaminophénol, le 2,5-diaminophénétol, la 4-méthylamino-, 3-amino-4-(2'-hydroxyéthyloxy)-, 3,4-méthylènediamino-, 3,4-méthylènedioxy-aniline, le 3-amino-2,4-dichloro-, 4-méthylamino-, 2-méthyl-5-amino-, 3-méthyl-4-amino-, 2-méthyl-5-(2-hydroxyéthylamino)-, 2-méthyl-5-amino-4-chloro-, 6-méthyl-3-amino-2-chloro-, 5-(2-hydroxyéthylamino)-4-méthoxy-2-méthyl-, 4-amino-2-aminométhyl-phénol, le 2-hydroxyméthyl-4-aminophénol, le 2-diéthylaminométhyl-4-aminophénol, le 2-diméthyl-aminométhyl-4-aminophénol, le 2,6-dichloro-4-aminophénol, le 1,3-diamino-2,4-diméthoxybenzène, l'acide 2-, 3-, 4-aminobenzoïque, -phénylacétique, l'acide 2,3-, 2,4-, 2,5-, 3,4-, 3,5-diaminobenzoïque, l'acide 4-, 5-aminosalicylique, l'acide 3-amino-4-hydroxy-, 4-amino-3-hydroxy-benzoïque, l'acide 2-, 3-, 4-aminobenzènesulfonique, l'acide 3-amino-4-hydroxybenzène-sulfonique, l'acide 4-amino-3-hydroxynaphtalène-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-, 1,2,4-triaminobenzène, le tétrachlorhydrate de 1,2,4,5-tétraaminobenzène, le trichlorhydrate de 2,4,5-triaminophénol, le pentachlorhydrate de pentaaminobenzène, l'hexachlorhydrate de hexaaminobenzène, le trichlorhydrate de 2,4,6-triaminorésorcinol, le sulfate de 4,5-diaminopyrocatéchol, le dichlorhydrate de 4,6-diaminopyrogallol, le sulfate de 3,5-diamino-4-hydroxypyrocatéchol, les nitriles aromatiques, les anilines en particulier les anilines contenant des groupes nitro, comme la 4-nitroaniline, la 4-nitro-1,3-phénylènediamine, le 2-nitro-4-amino-1-(2-hydroxyéthylamino)-benzène, le 2-nitro-1-amino-4-[bis-(2-hydroxyéthyl)-amino]-benzène, l'acide 4-amino-2-nitrodiphénylamine-2'-carboxylique, le 2-amino-6-chloro-4-nitrophénol, le 1-amino-5-chloro-4-(2-hydroxyéthylamino)-2-nitrobenzène, les anilines aromatiques ou les phénols ayant un radical aromatique supplémentaire comme le dichlorhydrate de 4,4'-diaminostilbène, l'acide 4,4'-diaminostilbène-2,2'-disulfonique, son sel de Na, l'acide 4,4'-diaminodiphénylméthane, son sulfure, son sulfoxyde, son amine, l'acide 4,4'-diaminodiphénylamine-2-sulfonique, la 4,4'-diaminobenzophénone, l'éther diphénylique, le tétrachlorhydrate de 3,3', 4,4'-tétraaminodiphényle, la 3,3', 4,4'-tétraamino-benzophénone, le tétrachlorhydrate de 1,3-bis-(2,4-diamino-phénoxy)-propane, le tétrachlorhydrate de 1,8-bis-(2,5-diaminophénoxy)-3,6-dioxaoctane, le 1,3-bis-(4-aminophénylamino)-propane, le 1,3-bis-(4-aminophénylamino)-2-propanol, le 1,3-bis-[N-(4-aminophényl)-2-hydroxyéthyl-amino]-2-propanol, le trichlorhydrate de N,N-bis-[2-(4-aminophénoxy)-éthyl]méthylamine,
les composés hétérocycliques azotés choisis dans le groupe formé par la 2-, 3-, 4-amino-, 2-amino-3-hydroxy-, 2,6-diamino-, 2,5-diamino-, 2,3-diamino-, 2-diméthylamino-5-amino, 3-amino-2-méthylamino-6-méthoxy-, 2,3-diamino-6-méthoxy-, 3,5-diamino-2,6-diméthoxy-, 2,4,5-triamino-, 2,6-dihydroxy-3,4-diméthyl-pyridine, la 4,5,6-triamino-, 2-hydroxy-4,5,6-triamino-, 4-hydroxy-2,5,6-triamino-, 2,4,5,6-tétraamino-, 2-méthylamino-4,5,6-triamino-, 2,4-, 4,5-diamino, 2-amino-4-méthoxy-6-méthyl-pyrimidine, le 2,3,4-triméthylpyrrole, le 2,4-diméthyl-3-éthyl-pyrrole, le 3,5-diaminopyrazole, le 3,5-diamino-1,2,4-triazole, le 3-amino-, 3-amino-5-hydroxypyrazole, la 2-, 3-, 8-amino-quinoléine, la 4-aminoquinaldine, l'acide 2-, 6-aminonicotinique, la 5-aminoisoquinoléine, le 5-, 6-aminoindazole, le 5-, 7-amino-benzimidazole, -benzothiazole, la 2,5-dihydroxy-4-morpholinoaniline, ainsi que les dérivés d'indole et d'indoline, comme le 4-, 5-, 6-, 7-aminoindole, le 4-, 5-, 6-, 7-hydroxyindole, le 5,6-dihydroxyindole, la 5,6-dihydroxyindoline et la 4-hydroxyindoline, ainsi que les sels acceptables sur le plan physiologique de ces composés, formés respectivement à partir d'acides de préférence inorganiques,
les composés hydroxyaromatiques choisis dans le groupe, formé par le 2-, 4-, 5-méthylrésorcinol, le 2,5-diméthylrésorcinol, le résorcinol, le 3-méthoxyphénol, le pyrocatéchol, l'hydroquinone, le pyrogallol, la phloroglucine, l'hydroxyhydroquinone, le 2-, 3-, 4-méthoxy-, 3-diméthylamino-, 2-(2-hydroxy-éthyl)-, 3,4-méthylènedioxy-phénol, l'acide 2,4-, 3,4-dihydroxy-benzoïque, -phénylacétique, l'acide gallique, l'acide 2,4,6-trihydroxybenzoïque, la 2,4,6-trihydroxyacétophénone, le 2-, 4-chlororésorcinol, le 1-naphtol, le 1,5-, 2,3-, 2,7-dihydroxynaphtalèlne, l'acide 6-diméthylamino-4-hydroxy-2-naphtalène-sulfonique, l'acide 3,6-dihydroxy-2,7-naphtalène-sulfonique, et
les composés à groupe CH actif choisis dans le groupe formé par l'iodure de 1,2,3,3,-tétraméthyl-3H-indolium, le p-toluène-sulfonate de 1,2,3,3-tétraméthyl-3H-indolium, le méthanesulfonate de 1,2,3,3-tétraméthyl-3H-indolium, la base de Fischer (la 1,3,3-triméthyl-2-méthylène-indoline), l'iodure de 2,3-diméthyl-benzothiazolium, le p-tolènesulfonate de 2,3-diméthyl-benzothiazolium, le p-toluènesulfonate de 1,2-diméthylnaphto[1,2-d]thiazolium, le p-toluène-sulfonate de 1-éthyl-2-méthylnapthto[1,2-d]thiazolium, la rhodanine, l'acide rhodanine-3-acétique, l'iodure de 1-éthyl-2-quinaldinium, l'iodure de 1,4-diméthylquinoléinium, l'iodure de 1-méthyl-2-quinaldinium, l'iodure de diméthylquinoléinium, l'acide barbiturique, l'acide thiobarbiturique, l'acide 1,3-diméthylthiobarbiturique, l'acide 1,3-diéthylthiobarbiturique, l'acide diéthylthiobarbiturique, l'oxindole, l'acétate de 3-indoxyle, la coumaranone, et la 1-méthyl-3-phényl-2-pyrazolinone.

6. Agent selon la revendication 5, **caractérisé en ce que** le composé supplémentaire est choisi dans le groupe formé par la N-(2-hydroxyéthyl)-N-éthyl-, 2-chloro-p-phénylène-diamine, la N,N-bis-(2-hydroxyéthyl)-p-phénylènediamine, le 4-aminophénol, la p-phénylènediamine, le 2-(2,5-diaminophényl)-éthanol, le 2,5-diaminotoluène, la 3,4-méthylènedioxyaniline, le 2-amino-4-(2-hydroxyéthylamino)-anisole, le 2-(2,4-diaminophénoxy)-éthanol, le 3-amino-2,4-dichloro-, 2-méthyl-5-amino-, 3-méthyl-4-amino-, 2-méthyl-5-(2-hydroxyéthylamino)-, 2-méthyl-5-amino-4-choro-, 6-méthyl-3-amino-2-chloro-, 2-aminométhyl-4-aminophénol, le 2-diéthylaminométhyl-4-aminophénol, le 2-diméthylaminométhyl-4-aminophénol, le 2,6-dichloro-4-aminophénol, le 2-hydroxyméthyl-4-aminophénol, le 3,4-méthylènedioxyphénol, l'acide 3,4-diaminobenzoïque, la 2,5-diamino-, 2-diméthylamino-5-amino-, 3-amino-2-méthylamino-6-méthoxy-, 2,3-diamino-6-méthoxy-, 3,5-diamino-2,6-diméthoxy-, 2,6-dihydroxy-3,4-diméthyl-pyridine, la 2-hydroxy-4,5,6-triamino-, 4-hydroxy-2,5,6-triamino-, 2,4,5,6-tétraamino-, 2-méthylamino-4,5-triaminopyrimidine, le 3,5-diaminopyrazole, le 3-amino-5-hydroxypyrazole, le 5,6-dihydroxyindole, et la 5,6-dihydroxyindoline, ainsi que respectivement les sels acceptables sur le plan physiologique de ces composés, formés à partir d'acides de préférence inorganiques.

7. Agent selon l'une quelconque des revendications 1 à 6, **caractérisé en ce qu'**il contient des colorants montant directement sur la fibre, issus du groupe des nitrophénylènediamines, des nitroaminophénols, des anthraquinones ou indophénols, de préférence en une quantité de 0,01 à 20% en poids, par rapport à la teinture totale.

8. Agent selon l'une quelconque des revendications 1 à 7, **caractérisé en ce que** des sels d'ammonium ou métalliques sont ajoutés, choisis dans le groupe des formiates, carbonates, halogénures, sulfates, butyrates, valériates, caproates, acétates, lactates, glycolates, tartrates, citrates, gluconates, propionates, phosphates et phosphonates, de métaux alcalins, comme le potassium, le sodium ou le lithium, de métaux alcalino-terreux, comme le magnésium, le calcium, le strontium ou le baryum, ou d'aluminium, de manganèse, de fer, de cobalt, de cuivre, ou de zinc.

9. Agent selon l'une quelconque des revendications 1 à 8, **caractérisé en ce qu'**il contient des agents d'oxydation, en particulier H₂O₂, en une quantité de 0,01 à 6% en poids par rapport à la solution d'application.

10. Agent selon l'une quelconque des revendications 1 à 9, **caractérisé en ce qu'**il contient des tensioactifs anioniques, zwitterioniques, ou non ioniques.

11. Utilisation d'aldéhydes ou de cétones aromatiques et/ou hétéroaromatiques de formule I 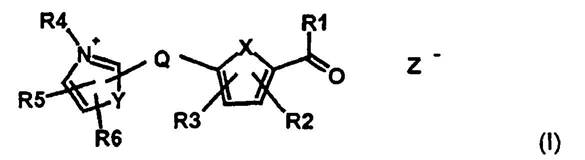 dans laquelle
R¹ représente un atome d'hydrogène, un groupe alkyle en C₁ à C₄, aryle ou hétéroaryle,
R², R³ représentent un atome d'hydrogène, un atome d'halogène, un groupe alkyle en C₁ à C₄, alcoxy en C₁ à C₄, ou hydroxyalcoxy en C₁ à C₄, un groupe hydroxy, nitro, amino, qui peut être substitué par des groupes alkyle en C₁ à C₄, qui peuvent former conjointement avec l'atome d'azote, un cycle hétérocyclique, les deux radicaux R² et R³ pouvant former également conjointement un cycle aromatique condensé, R⁴ représente un groupe alkyle en C₁ à C₄, alcényle en C₁ à C₄, aryle, aralkyle, carboxyalkyle en C₁ à C₄, ou sulfoalkyle en C₁ à C₄,
R⁵, R⁶ représentent un atome d'hydrogène, un atome d'halogène, un groupe alkyle en C₁ à C₄, alcoxy en C₁ à C₄, hydroxyalcoxy en C₁ à C₄, un groupe hydroxy, nitro, amino, qui peut être substitué par des groupes alkyle en C₁ à C₄, qui peuvent former conjointement avec l'atome d'azote, un cycle hétérocyclique, les deux radicaux R⁵ et R⁶ pouvant former également conjointement un cycle aromatique condensé,
Q désigne un groupe méthylène ou vinylène,
Y et X désignent un atome d'azote substitué par un groupe aralkyle ou aryle, un atome d'oxygène, un atome de soufre, ou un groupe vinylène éventuellement substitué, et
Z- désigne un anion tel qu'un ion halogènure, sulfate d'alkyle en C₁ à C₄, alcane-sulfonate en C₁ à C₄, arènesulfonate, perfluoroalcane-sulfate en C₁ à C₄, perhalogénate, sulfate, hydrogénosulfate, carboxylate, tétrafluoroborate, hexafluorophosphate ou tétrachlorozincate,
la liaison à l'hétérocycle quaternisé étant fixée de préférence dans la position 2 et pour Y = vinylène, également dans la position 4,
en tant que composant colorant dans des teintures capillaires par oxydation.

12. Procédé pour colorer les fibres kératiniques, en particulier les cheveux humains, dans lequel une teinture, contenant
A) au moins un aldéhyde ou une cétone aromatique et/ou hétéroaromatique de formule I, 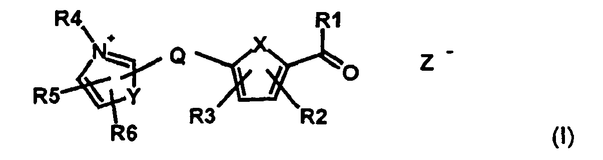 dans laquelle
R¹ représente un atome d'hydrogène, un groupe alkyle en C₁ à C₄, aryle ou hétéroaryle,
R², R³ représentent un atome d'hydrogène, un atome d'halogène, un groupe alkyle en C₁ à C₄, alcoxy en C₁ à C₄, ou hydroxyalcoxy en C₁ à C₄, un groupe hydroxy, nitro, amino, qui peut être substitué par des groupes alkyle en C₁ à C₄, qui peuvent former conjointement avec l'atome d'azote, un cycle hétérocyclique, les deux radicaux R² et R³ pouvant former également conjointement un cycle aromatique condensé, R⁴ représente un groupe alkyle en C₁ à C₄, alcényle en C₁ à C₄, aryle, aralkyle, carboxyalkyle en C₁ à C₄, ou sulfoalkyle en C₁ à C₄,
R⁵, R⁶ représentent un atome d'hydrogène, un atome d'halogène, un groupe alkyle en C₁ à C₄, alcoxy en C₁ à C₄, ou hydroxyalcoxy en C₁ à C₄, un groupe hydroxy, nitro, amino, qui peut être substitué par des groupes alkyle en C₁ à C₄, qui peuvent former conjointement avec l'atome d'azote, un cycle hétérocyclique, les deux radicaux R⁵ et R⁶ pouvant former également conjointement un cycle aromatique condensé,
Q désigne un groupe méthylène ou vinylène,
Y et X désignent un atome d'azote substitué par un groupe aralkyle ou aryle, un atome d'oxygène, un atome de soufre, ou un groupe vinylène éventuellement substitué, et
Z⁻ désigne un anion tel qu'un ion halogènure, sulfate d'alkyle en C₁ à C₄, alcane-sulfonate en C₁ à C₄, arènesulfonate, perfluoroalcanesulfate en C₁ à C₄, perhalogénate, sulfate, hydrogénosulfate, carboxylate, tétrafluoroborate, hexafluorophosphate ou tétrachlorozincate,
la liaison à l'hétérocycle quaternisé étant fixée de préférence dans la position 2 et pour Y = vinylène, également dans la position 4,
B) au moins un composé avec un groupe amino primaire ou secondaire ou un groupe hydroxy, chosi parmi les amines aromatiques primaires ou secondaires, les composés hétérocycliques azotés et les composés hydroxyaromatiques, et/ou au moins un composé à groupe CH actif, ainsi que des ingrédients cosmétiques courants, est appliquée sur les fibres kératiniques pendant quelque temps, habituellement pendant environ 30 minutes, et laissée sur la fibre, puis rincée à nouveau ou éliminée par lavage avec un shampoing.
